# EUROPÄISCHE PATENTSCHRIFT

(11) **EP 3 798 611 B1**
(45) Veröffentlichungstag und Bekanntmachung des Hinweises auf die Patenterteilung: **03.05.2023**
(21) Anmeldenummer: 19200607.0
(22) Anmeldetag: 30.09.2019
(51) Int. Cl.: G01N 21/39, G01N 21/3504, G01J 3/433, G01J 3/02, H01S 5/062, H01S 5/0687

(54) **VERFAHREN UND GASANALYSATOR ZUR MESSUNG DER KONZENTRATION EINER GASKOMPONENTE IN EINEM MESSGAS**
METHOD AND GAS ANALYSER FOR MEASURING THE CONCENTRATION OF A GAS COMPONENT IN A GAS TO BE MEASURED
PROCÉDÉ ET ANALYSEUR DE GAZ PERMETTANT DE MESURER LA CONCENTRATION D'UN COMPOSANT GAZEUX DANS UN GAZ MESURÉ

(43) Veröffentlichungstag der Anmeldung: 31.03.2021
(73) Patentinhaber: Siemens Aktiengesellschaft, 80333 München (DE)
(72) Erfinder: Steinbacher, Franz, 76137 Karlsruhe (DE)
(74) Vertreter: Siemens Patent Attorneys

(56) Entgegenhaltungen:
- EP-A1- 3 001 180
- EP-A1- 3 339 839
- US-A- 6 064 488
- US-A1- 2005 046 852
- US-A1- 2014 361 172
- US-B1- 6 341 521
- JINGJING WANG ET AL: "High-sensitivity off-axis integrated cavity output spectroscopy implementing wavelength modulation and white noise perturbation", OPTICS LETTERS, Bd. 44, Nr. 13, 1. Juli 2019 (2019-07-01), Seite 3298, XP055686627, US ISSN: 0146-9592, DOI: 10.1364/OL.44.003298

## Beschreibung

Die Erfindung betrifft ein Verfahren zur Messung der Konzentration einer Gaskomponente in einem Messgas.

Sie betrifft ferner einen Gasanalysator zur Messung der Konzentration der Gaskomponente in dem Messgas.

Laserspektrometer werden insbesondere für die optische Gasanalyse in der Prozessmesstechnik eingesetzt. Dabei erzeugt eine Laserdiode Licht im Infrarotbereich, das entlang einer Messstrecke in einer Prozessanlage oder einer Gaszelle durch ein Prozessgas (Messgas) geführt und anschließend detektiert wird. Die Wellenlänge des Lichts wird auf eine spezifische Absorptionslinie der jeweils zu messenden Gaskomponente abgestimmt, wobei die Laserdiode die Absorptionslinie periodisch wellenlängenabhängig abtastet. Dazu wird die Laserdiode innerhalb von aufeinanderfolgenden Abtastintervallen mit einem rampen- oder dreieckförmigen Stromsignal angesteuert. Bei der direkten Absorptionsspektroskopie (DAS) kann die Konzentration der interessierenden Gaskomponente unmittelbar aus der an der Stelle der Absorptionslinie detektierten Minderung der Lichtintensität (Absorption) bestimmt werden. Bei der Wellenlängenmodulationsspektroskopie (WMS) wird während der vergleichsweise langsamen wellenlängenabhängigen Abtastung der Absorptionslinie zusätzlich die Wellenlänge des erzeugten Lichts mit einer Frequenz im kHz-Bereich und kleiner Amplitude sinusförmig moduliert. Da das Profil der Absorptionslinie nicht linear ist, werden auch Harmonische höherer Ordnung in dem Detektor- oder Messsignal erzeugt. Das Messsignal wird üblicherweise bei einer n-ten Oberschwingung, vorzugsweise der zweiten Harmonischen, durch phasensensitive Lock-in Technik demoduliert und für jedes Abtastintervall zu einem die Konzentration der zu messenden Gaskomponente beinhaltenden Messergebnis ausgewertet.

Die Nachweis- und Bestimmungsgrenze für die Messung der Konzentration der Gaskomponente wird durch Rauschen begrenzt, welches dem Messsignal überlagert. Das Rauschen setzt sich hauptsächlich aus dem Rauschen des Gasanalysators auf der Laser- und der Detektorseite sowie dem Rauschen aus der Messstrecke (verursacht durch Turbulenzen, Partikel) zusammen.

Aus der WO 2016/050577 A1 oder der DE 10 2015 207 192 A1 ist es bekannt, den Strom zur Ansteuerung der Laserdiode zusätzlich mit einer Hochfrequenz (HF) zu modulieren, die in Abhängigkeit von den Eigenschaften der Laserdiode so hoch gewählt ist, dass keine Wellenlängenmodulation des erzeugten Lichts stattfindet. Das Detektor- bzw. Messsignal wird bei der Frequenz der zusätzlichen HF-Modulation demoduliert, und das dabei erhaltene demodulierte Messsignal wird zur Erzeugung des Messergebnisses ausgewertet.

Da die Wellenlänge des Lichts über die innere Temperatur der Laserdiode eingestellt bzw. verändert wird, und diese innere Temperatur wiederum über die Verlustleistung durch den Laserstrom und über die Umgebungstemperatur einstellbar bzw. veränderbar ist, kann eine Wellenlängenmodulation nur mit niedrigen Modulationsfrequenzen, maximal im kHz-Bereich, erfolgen. Bei höheren Frequenzen im MHz-Bereich wird dagegen nur noch die Intensität des Lichts, nicht aber seine Wellenlänge, moduliert. Durch die HF-Modulation wird das Basisband des auszuwertenden Messsignals aus dem durch das Rauschen des Gasanalysators und der Messstrecke gestörten Frequenzbereich nahe DC in einen hohen Frequenzbereich kopiert, in dem dieses Rauschen nicht mehr vorhanden ist.

Da die HF-Modulation nur bis zur Aussteuergrenze der Laserdiode erfolgen kann, wird in der DE 10 2015 207 192 A1 vorgeschlagen, die HF-Modulation mit einem Spreizcode zu multiplizieren, so dass sich die Signalenergie der HF-Modulation auf ein breites Frequenzspektrum verteilt und daher eine höhere auswertbare Gesamtenergie zur Verfügung steht. Detektorseitig wird das demodulierte Messsignal durch Korrelation mit dem Spreizcode korreliert und das erhaltene Korrelationssignal ausgewertet. Durch die Korrelation des demodulierten Messsignals mit dem Spreizcode wird außerdem das in dem Messsignal enthaltene Rauschen des Gasanalysators reduziert, weil das Rauschen mit dem Spreizcode nicht korreliert.

Jingjing Wang et al: "High-sensitivity off-axis integrated cavity output spectroscopy implementing wavelength modulation and white noise perturbation", Optics Letters, Bd. 44, Nr. 13, 1. Juli 2019, Seite 3298, offenbart ein off-axis-Integrated-Cavity-Output-Spektrometer (OA-ICOS), bei dem eine das Messgas enthaltende Gaszelle als optischer Resonator ausgebildet ist, in den der Laserstrahl versetzt und verkippt zur optischen Achse eingekoppelt wird und daher eine Vielzahl von Moden des Resonators anregt. Der zur Wellenlängenabtastung rampenförmig und zur Wellenlängenmodulation sinusförmig modulierte Strom der Laserdiode wird zusätzlich mit einem weißen Rauschen moduliert, um das Rauschen durch Fluktuationen der Resonatormoden (residual cavity-mode fluctuation) zu unterdrücken. Die Einkopplung des weißen Rauschens führt jedoch auch zu einer Verbreiterung der Absorptionslinien und einer Verringerung ihrer Höhe, so dass ein Kompromiss zwischen einer effizienten Unterdrückung des Resonatormoden-Rauschens und einer möglichst geringen Verformung der Spektrallinienform gefunden werden muss.

Die US 2005/046852 A1 offenbart ein WMS-Laserspektrometer mit einem Strahlteiler, der einen Teil des von der Laserdiode erzeugten Lichts durch das Messgas auf einen Messdetektor und den anderen Teil auf einen Monitordetektor führt. Die den Strom für die Laserdiode erzeugende Stromquelle wird in regelmäßigen Abständen außerhalb der Abtastperioden für die wellenlängenabhängige Abtastung der interessierenden Absorptionslinien mit einem Burst-Signal angesteuert. Dieser Burst wird sowohl in dem Messsignal als auch in dem Monitorsignal detektiert und zur Normalisierung der Messung verwendet.

Die EP 3 339 839 A1 offenbart ein WMS-Laserspektrometer mit einem Strahlteiler, der einen Teil des von der Laserdiode erzeugten Lichts durch das Messgas und den anderen Teil durch ein Referenzgas auf einen Referenzdetektor führt, der das Absorptionsspektrum des Referenzgases detektiert. Zur Ermittlung der Konzentration der interessierenden Gaskomponente des Messgases wird das separat detektierte Absorptionsspektrum des Referenzgases von dem des Messgases subtrahiert.

Der Erfindung liegt die Aufgabe zugrunde, das Messsignal-Rausch-Verhältnis weiter zu verbessern.

Gemäß der Erfindung wird die Aufgabe durch das in Anspruch 1 definierte Verfahren sowie den in Anspruch 5 angegebenen Gasanalysator gelöst.

Vorteilhafte Weiterbildungen der Erfindung sind in den Unteransprüchen angegeben.

In Bezug auf das Verfahren sieht also die Erfindung vor, dass zur Messung der Konzentration einer Gaskomponente in einem Messgas mittels eines Gasanalysators,
- eine wellenlängenabstimmbare Laserdiode mit einem Strom angesteuert wird,
- ein Teil des von der Laserdiode erzeugten Lichts durch das Messgas auf einen Messdetektor geführt wird, der ein Messsignal erzeugt,
- der andere Teil des Lichts auf einen Monitordetektor geführt wird, der ein Monitorsignal erzeugt,
- der Strom in periodisch aufeinanderfolgenden Abtastintervallen variiert wird, um eine interessierende Absorptionslinie der Gaskomponente wellenlängenabhängig abzutasten,
- der Strom ferner mit einem hochfrequenten Rauschsignal moduliert wird, dessen untere Grenzfrequenz in Abhängigkeit von den Eigenschaften der Laserdiode so hoch gewählt ist, dass keine Wellenlängenmodulation stattfindet, und
- das Messsignal mit dem Monitorsignal zur Erzeugung eines Korrelationssignals korreliert wird und anschließend das Korrelationssignal zur Erzeugung eines Messergebnisses ausgewertet wird.

Im Unterschied zu dem aus der DE 10 2015 207 192 A1 bekannten Verfahren, wird der Strom für die Laserdiode ohne einen HF-Träger direkt mit einem hochfrequenten Rauschen moduliert. Das aus dem Licht der Laserdiode nach Durchstrahlen des Messgases erhaltene Messsignal wird mit dem unmittelbar aus dem Licht der Laserdiode erzeugten Monitorsignal korreliert. Das auf der Laserseite des Gasanalysators vorhandene Rauschen der Laserdiode, der Treiberelektronik usw. wird dadurch Bestandteil der Modulation mit dem hochfrequenten Rauschsignal, so dass sich das Rauschen des Messignals von dem Rauschen des Monitorsignals nur durch die durch die Absorption in dem Messgas hervorgerufenen Einflüsse unterscheidet.

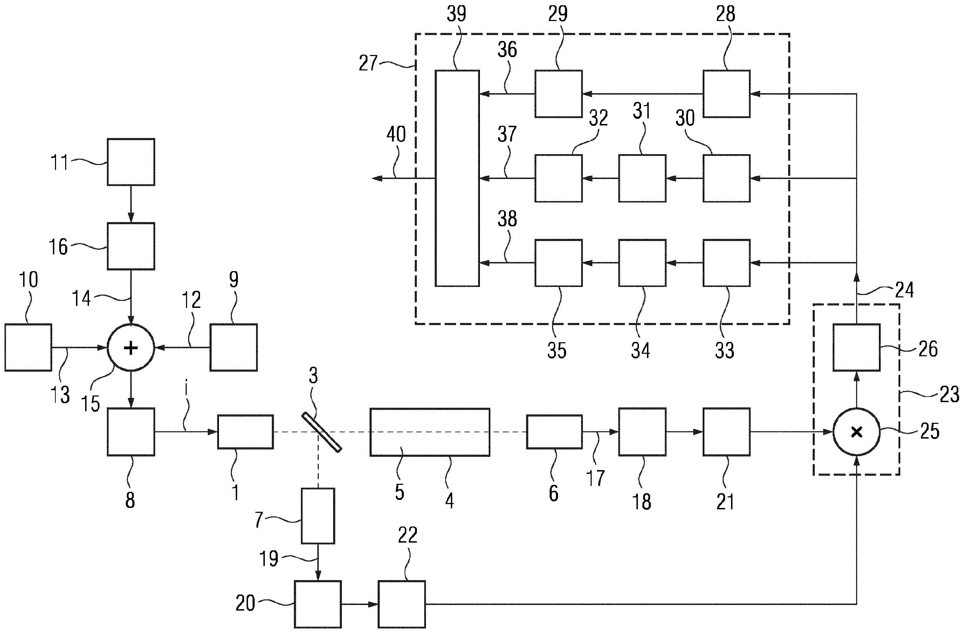

Zur weiteren Erläuterung der Erfindung wird im Folgenden auf die Figur der Zeichnung Bezug genommen, die beispielhaft ein stark vereinfachtes Blockschaltbild des erfindungsgemäßen Gasanalysators zeigt.

Der Gasanalysator enthält eine Laserdiode 1, deren Licht 2 über einen Strahlteiler 3 zu einem Teil durch ein in einem Messvolumen 4, beispielsweise einer Gaszelle oder einer Prozessgasleitung, enthaltenes Messgas 5 auf einen Messdetektor 6 und zu dem anderen Teil auf einen Monitordetektor 7 geführt ist. Die Laserdiode 1 wird von einer steuerbaren Stromquelle (Treiber) 8 mit einem Injektionsstrom i angesteuert, wobei die Intensität und die Wellenlänge des erzeugten Lichts 2 von dem Strom i und der Betriebstemperatur der Laserdiode 1 abhängen. Zur Variation und Modulation des Stromes i erzeugen ein Signalgenerator 9, eine optionale Niederfrequenz-(NF-)Modulationseinrichtung 10 und ein Rauschsignalgenerator 11 unterschiedliche Signale 12, 13, 14, die der Stromquelle 8 über einen Summierer 15 zugeführt werden.

Mit dem Signal 12 des Signalgenerators 9 wird der Strom i periodisch entsprechend einer vorgegebenen, vorzugsweise rampen- oder dreieckförmigen Funktion variiert, um mit der mehr oder weniger linear folgenden Wellenlänge des erzeugten Lichts 2 mindestens eine ausgewählte Absorptionslinie einer interessierenden Gaskomponente in dem Messgas 5 wellenlängenabhängig abzutasten. Das Signal 12 kann zusätzlich Bursts enthalten, die in regelmäßigen Abständen, z. B. nach jeder Abtastperiode, aufeinander folgen und während derer die Laserdiode 1 mit einer Burstfrequenz von beispielsweise 3 kHz ein- und ausgeschaltet wird, um später eine Normalisierung der Messung ermöglichen.

Die Niederfrequenz-(NF-)Modulationseinrichtung 10 ist vorgesehen, wenn die Konzentration der interessierenden Gaskomponente auf Basis der Wellenlängenmodulationsspektroskopie (WMS) bestimmt werden soll. In diesem Fall werden der Strom i und damit die Wellenlänge des erzeugten Lichts 2 mit einer Frequenz f im kHz-Bereich (z. B. < 100 kHz) und kleiner Amplitude sinusförmig moduliert.

Bei dem von dem Rauschsignalgenerator 11 erzeugten und der steuerbaren Stromquelle 8 zugeführten Signal 14 handelt es sich um ein hochfrequenten Rauschsignal, dessen untere Grenzfrequenz in Abhängigkeit von den Eigenschaften der Laserdiode 1 mit beispielsweise 5 MHz bis 100 MHz so hoch gewählt bzw. hier z. B. durch ein Hochpassfilter 16 bestimmt ist, dass keine Wellenlängenmodulation stattfindet und nur die Intensität des erzeugten Lichts 2 moduliert wird.

Der Messdetektor 6 erzeugt in Abhängigkeit von der detektierten Lichtintensität ein Messsignal 17, das in einer Messsignal-Verarbeitungsstufe 18 verstärkt und digitalisiert wird. Ebenso erzeugt der Monitordetektor 7 ein Monitorsignal 19, das in einer Monitorsignal-Verarbeitungsstufe 20 verstärkt und digitalisiert wird. Das digitalisierte Messsignal 17 und das digitalisierte Monitorsignal 19 werden jeweils in Filtern 21 bzw. 22 mit hier z. B. der unteren Grenzfrequenz des Rauschsignals 14 hochpassgefiltert, bevor sie einem Korrelator 23 zugeführt werden, der das Messsignal 17 mit dem Monitorsignal 19 korreliert und ein Korrelationssignal 24 erzeugt. Durch die Hochpassfilterung werden die Signalanteile der rampen- bzw. dreieckförmigen Modulation sowie der NF-Modulation von dem Korrelator 23 zurückgehalten, so dass die Korrelation des Messsignals 17 mit dem Monitorsignal 19 nur für das in ihnen enthaltene Rauschen erfolgt. Bei dem gezeigten Ausführungsbeispiel sind die beiden Signalverarbeitungspfade für das Messsignal 17 und das Monitorsignal 19 identisch bzw. enthalten baugleiche Elektroniken, so dass die Verzögerungen der beiden Signale 17, 19 gleich sind und der Korrelator 23 auf einfache Weise in Form eines Multiplizierers 25 mit einem nachgeordneten Tiefpass (Mittelwertbildner, Integrator) 26 ausgebildet sein kann. Da das Korrelationsmaximum immer bei Deckungsgleichheit der korrelierten Signale auftritt und sich das Rauschen permanent ändert, dürfen die beiden Signale 17, 19 keinen zeitlichen Versatz zueinander haben. Sind daher z. B. bei einem In-situ-Gasanalysator mit an gegenüberliegenden Seiten eines Rauchgaskanals montierten Messköpfen der Monitordetektor 19 zusammen mit der Laserdiode 1 in dem einen Messkopf und der Messdetektor 17 in dem anderen Messkopf angeordnet, müssen Laufzeitverzögerungen aufgrund unterschiedlich langer Signalwege zwischen den Detektoren 6, 7 und dem Korrelator 23 durch Zwischenspeichern ausgeglichen werden.

Im Weiteren wird das Korrelationssignal 24 in einer Auswerteeinrichtung 27 auf Basis der direkten Absorptionsspektroskopie (DAS) und/oder der Wellenlängenmodulationsspektroskopie (WMS) ausgewertet.

Für die DAS-Auswertung durchläuft das Korrelationssignal 24 ein Tiefpassfilter 28, bevor es in einer Recheneinrichtung 29 ausgewertet und normiert wird. Die Grenzfrequenz des Tiefpassfilters 28 ist so gewählt, dass sie höher als die Frequenz ist, mit der die Intensität des Lichts 2 bei der mittels des rampen- oder dreieckförmigen Signals 12 erzielten wellenlängenabhängigen Abtastung der ausgewählten Absorptionslinie der interessierenden Gaskomponente in dem Messgas 5 variiert. Bei dem hier gezeigten Beispiel beträgt die Grenzfrequenz des Tiefpassfilters 28 beispielsweise 3 kHz.

Für eine WMS-Auswertung bei der einfachen NF-Modulationsfrequenz f wird das Korrelationssignal 24 nach Durchlaufen eines Bandpassfilters 30 mit der Mittenfrequenz f in einem Lock-in-Detektor 31 bei der Frequenz 2f phasensensitiv detektiert und anschließend in einer Recheneinrichtung 32 ausgewertet.

Für eine WMS-Auswertung bei der zweifachen (oder n-fachen) NF-Modulationsfrequenz f wird das Korrelationssignal 24 nach Durchlaufen eines Bandpassfilters 33 mit der Mittenfrequenz 2f (oder nf) in einem Lock-in-Detektor 34 bei der Frequenz 2f (oder nf) phasensensitiv detektiert und anschließend in einer Recheneinrichtung 35 ausgewertet.

Bei dem hier gezeigten Beispiel beträgt die Bandbreite der Bandpassfilter 30, 33 beispielsweise 3 kHz.

Die Ergebnisse 36, 37, 38 der DAS- und WMS-Auswertungen werden in einer weiteren Recheneinrichtung 39 zusammengeführt, um schließlich als Messergebnis 40 die Konzentration der zu messenden Gaskomponente zu erhalten.

## Patentansprüche

1. Verfahren zur Messung der Konzentration einer Gaskomponente in einem Messgas (5) mittels eines Gasanalysators, wobei
- eine wellenlängenabstimmbare Laserdiode (1) mit einem Strom (i) angesteuert wird,
- ein Teil des von der Laserdiode (1) erzeugten Lichts (2) durch das Messgas (1) auf einen Messdetektor (6) geführt wird, der ein Messsignal (17) erzeugt,
- der andere Teil des Lichts (2) auf einen Monitordetektor (7) geführt wird, der ein Monitorsignal (19) erzeugt,
- der Strom (i) in periodisch aufeinanderfolgenden Abtastintervallen variiert wird, um eine interessierende Absorptionslinie der Gaskomponente wellenlängenabhängig abzutasten,
- der Strom (i) ferner mit einem hochfrequenten Rauschsignal (14) moduliert wird, dessen untere Grenzfrequenz in Abhängigkeit von den Eigenschaften der Laserdiode (1) so hoch gewählt ist, dass keine Wellenlängenmodulation stattfindet, und
- das Messsignal (17) mit dem Monitorsignal (19) zur Erzeugung eines Korrelationssignals (24) korreliert wird und anschließend das Korrelationssignal (24) zur Erzeugung eines Messergebnisses (40) ausgewertet wird.

2. Verfahren nach Anspruch 1, **dadurch gekennzeichnet, dass** das Messsignal (17) und das Monitorsignal (19) vor der Korrelation mit einer Grenzfrequenz kleiner als die des hochfrequenten Rauschsignals (14) hochpassgefiltert werden.

3. Verfahren nach Anspruch 1 oder 2 basierend auf der direkten Absorptionsspektroskopie.

4. Verfahren nach einem der vorangehenden Ansprüche basierend auf der Wellenlängenmodulationsspektroskopie wobei der Strom (i) der Laserdiode (1) zusätzlich mit geringer Amplitude und mindestens einer zusätzlichen Frequenz moduliert wird, die so niedrig gewählt ist, dass eine Wellenlängenmodulation stattfindet.

5. Gasanalysator zur Messung der Konzentration einer Gaskomponente in einem Messgas (5) mit
- einer wellenlängenabstimmbaren Laserdiode (1),
- einer die Laserdiode (1) mit einem Strom (i) speisenden Stromquelle (8),
- einem Signalgenerator (9), der die Stromquelle (8) steuert, um den Strom (i) zur wellenlängenabhängigen Abtastung einer interessierenden Absorptionslinie der Gaskomponente in periodisch aufeinanderfolgenden Abtastintervallen zu variieren,
- einem Rauschsignalgenerator (11), der die Stromquelle (6) steuert, um den Strom (i) mit einem hochfrequenten Rauschsignal (14) zu modulieren, dessen untere Grenzfrequenz in Abhängigkeit von den Eigenschaften der Laserdiode (1) so hoch gewählt ist, dass keine Wellenlängenmodulation stattfindet,
- einem ein Messsignal (17) erzeugenden Messdetektor (6),
- einem ein Monitorsignal (19) erzeugenden Monitordetektor (7),
- einem Strahlteiler (3), der einen Teil des von der Laserdiode (1) erzeugten Lichts (2) durch das Messgas (5) auf den Messdetektor (6) und den anderen Teil auf den Monitordetektor (7) führt,
- einem Korrelator (23), der das Messsignal (17) mit dem Monitorsignal (19) korreliert und ein Korrelationssignal (24) erzeugt, und
- einer Auswerteeinrichtung (27), die das Korrelationssignal (24) zur Erzeugung eines Messergebnisses (40) auswertet.

6. Gasanalysator nach Anspruch 5, **dadurch gekennzeichnet, dass** der Korrelator (23) einen Multiplizierer (25) mit einem nachgeordneten Tiefpass/Mittelwertbildener/Integrator (26) aufweist.

7. Gasanalysator nach Anspruch 5 oder 6, **dadurch gekennzeichnet, dass** dem Korrelator (23) Hochpassfilter (21, 22) vorgeordnet sind, die das Messsignal (17) und das Monitorsignal (19) vor der Korrelation mit einer Grenzfrequenz kleiner als die des hochfrequenten Rauschsignals (14) hochpassfiltern.

8. Gasanalysator nach einem der Ansprüche 5 bis 7, **dadurch gekennzeichnet, dass** die Auswerteeinrichtung (27) dazu ausgebildet ist, das Korrelationssignal (24) auf der Grundlage der direkten Absorptionsspektroskopie auszuwerten.

9. Gasanalysator nach einem der Ansprüche 5 bis 8, **dadurch gekennzeichnet,**
- **dass** eine Niederfrequenz-(NF-)Modulationseinrichtung (10) vorhanden ist, die die Stromquelle (8) steuert um den Strom (i) zusätzlich mit geringer Amplitude und mindestens einer zusätzlichen Frequenz zu modulieren, die so niedrig gewählt ist, dass eine Wellenlängenmodulation stattfindet, und
- **dass** die Auswerteeinrichtung (27) dazu ausgebildet ist, das Korrelationssignal (24) auf der Grundlage der Wellenlängenmodulationsspektroskopie auszuwerten.

## Claims

1. Method for measuring the concentration of a gas component in a measurement gas (5) by means of a gas analyser, wherein
- a wavelength-tunable laser diode (1) is actuated with a current (i),
- one part of the light (2) generated by the laser diode (1) is guided through the measurement gas (1) to a measuring detector (6), which generates a measuring signal (17),
- the other part of the light (2) is guided to a monitor detector (7), which generates a monitor signal (19),
- the current (i) is varied in periodically consecutive scanning intervals in order to scan an absorption line of interest of the gas component as a function of the wavelength,
- the current (i) is further modulated with a radio-frequency noise signal (14), the lower cut-off frequency of which is selected as a function of the properties of the laser diode (1) high enough to ensure that no wavelength modulation occurs and
- the measuring signal (17) is correlated with the monitor signal (19) to generate a correlation signal (24) and then the correlation signal (24) is evaluated to generate a measurement result (40).

2. Method according to claim 1, **characterised in that** the measuring signal (17) and, before the correlation, the monitor signal (19) is high-pass filtered with a cut-off frequency lower than that of the radio-frequency noise signal (14).

3. Method according to claim 1 or 2 based on direct absorption spectroscopy.

4. Method according to one of the preceding claims based on wavelength modulation spectroscopy, wherein the current (i) of the laser diode (1) is additionally modulated with a lower amplitude and at least one additional frequency, which is selected low enough to ensure that wavelength modulation occurs.

5. Gas analyser for measuring the concentration of a gas component in a measurement gas (5) with
- a wavelength-tunable laser diode (1),
- a current source (8) that feeds the laser diode (1) with a current (i),
- a signal generator (9), which controls the current source (8) in order to vary the current (i) for wavelength-dependent scanning of an absorption line of interest of the gas component in periodically consecutive scanning intervals,
- a noise-signal generator (11), which controls the current source (6) in order to modulate the current (i) with a radio-frequency noise signal (14), the lower cut-off frequency of which is selected as a function of the properties of the laser diode (1) high enough to ensure that no wavelength modulation occurs,
- a measuring detector (6) that generates a measuring signal (17),
- a monitor detector (7) that generates a monitor signal (19),
- a beam splitter (3), which guides one part of the light (2) generated by the laser diode (1) through the measurement gas (5) to the measuring detector (6) and the other part to the monitor detector (7),
- a correlator (23), which correlates the measuring signal (17) with the monitor signal (19) and generates a correlation signal (24) and
- an evaluation device (27), which evaluates the correlation signal (24) in order to generate a measurement result (40).

6. Gas analyser according to claim 5, **characterised in that** the correlator (23) comprises a multiplier (25) with a downstream low pass/averaging unit/integrator (26).

7. Gas analyser according to claim 5 or 6, **characterised in that** high-pass filters (21, 22) arranged upstream of the correlator (23) high-pass filter the measuring signal (17) and the monitor signal (19) before correlation with a cut-off frequency lower than that of the radio-frequency noise signal (14) .

8. Gas analyser according to one of claims 5 to 7, **characterised in that** the evaluation device (27) is embodied to evaluate the correlation signal (24) based on direct absorption spectroscopy.

9. Gas analyser according to one of claims 5 to 8, **characterised in**
- **that** a low-frequency (LF) modulation device (10) is provided, which controls the current source (8) in order to modulate the current (i) additionally with a lower amplitude and at least one additional frequency, which is selected low enough to ensure that wavelength modulation occurs and
- **that** the evaluation device (27) is embodied to evaluate the correlation signal (24) based on wavelength modulation spectroscopy.

## Revendications

1. Procédé de mesure de la concentration d'un constituant gazeux d'un gaz (5) à mesurer au moyen d'un analyseur de gaz, dans lequel
- on excite, par un courant (i), une diode (1) laser accordable en longueur d'onde,
- on envoie une partie de la lumière (2) produite par la diode (1) laser à travers le gaz (1) à mesurer sur un détecteur (6) de mesure, qui produit un signal (17) de mesure,
- on envoie l'autre partie de la lumière (2) sur un détecteur (7) de moniteur, qui produit un signal (19) de moniteur,
- on fait varier le courant (i) dans des intervalles d'échantillonnage se succédant périodiquement pour échantillonner, en fonction de la longueur d'onde, une raie d'absorption intéressante du constituant gazeux,
- on module le courant (i) en outre par un signal (14) de bruit de haute fréquence, dont la fréquence limite inférieure est, en fonction des propriétés de la diode (1) laser, choisie si haute qu'une modulation en longueur d'onde n'a pas lieu, et
- on met le signal (17) de mesure en corrélation avec le signal (19) de moniteur pour la production d'un signal (24) de corrélation, et on analyse ensuite le signal (24) de corrélation pour la production d'un résultat (40) de mesure.

2. Procédé suivant la revendication 1, **caractérisé en ce qu'**on filtre passe haut le signal (17) de mesure et le signal (19) de moniteur avant la corrélation à une fréquence limite plus petite que le signal (14) de bruit de haute fréquence.

3. Procédé suivant la revendication 1 ou 2, reposant la spectroscopie d'absorption directe.

4. Procédé suivant l'une des revendications précédentes, reposant sur la spectroscopie de modulation de longueur d'onde, dans lequel on module le courant (i) de la diode (1) laser supplémentairement avec une amplitude petite et au moins une fréquence supplémentaire, qui est choisie si basse qu'une modulation en longueur d'onde a lieu.

5. Analyseur de gaz pour la mesure de la concentration d'un constituant gazeux d'un gaz (5) à mesurer, comprenant
- une diode (1) laser accordable en longueur d'onde,
- une source (8) de courant alimentant la diode (1) laser en un courant (i),
- un générateur (9) de signal, qui commande la source (8) de courant, afin de faire varier, dans des intervalles d'échantillonnages se succédant périodiquement, le courant (i) pour l'échantillonnage en fonction de la longueur d'onde d'une raie d'absorption intéressante du constituant gazeux,
- un générateur (11) de signal de bruit, qui commande la source (6) de courant, afin de moduler le courant (i) par un signal (14) de bruit de haute fréquence, dont la fréquence limite inférieure est, en fonction des propriétés de la diode (1) laser, choisie si haute qu'une modulation en longueur d'onde n'a pas lieu,
- un détecteur (6) de mesure produisant un signal (17) de mesure,
- un détecteur (7) de moniteur produisant un signal (19) de moniteur,
- un diviseur (3) de faisceau, qui renvoie une partie de la lumière (2), produite par la diode (1) laser, à travers le gaz (5) à mesurer sur le détecteur (6) de mesure et l'autre partie sur le détecteur (7) de moniteur,
- un corrélateur (23), qui met le signal (17) de mesure en corrélation avec le signal (19) de moniteur et qui produit un signal (24) de corrélation, et
- un dispositif (27) d'analyse, qui analyse le signal (24) de corrélation pour la production d'un résultat (40) de mesure.

6. Analyseur de gaz suivant la revendication 5, **caractérisé en ce que** le corrélateur (23) a un multiplicateur (25) ayant un passe bas / formeur de valeur moyenne / intégrateur (26) en aval.

7. Analyseur de gaz suivant la revendication 5 ou 6, **caractérisé en ce qu'**il est monté, en amont du corrélateur (23), des filtres (21, 22) passe haut, qui filtrent passe haut le signal (17) de mesure et le signal (19) de moniteur, avant la corrélation à une fréquence limite plus petite que celle du signal (14) de bruit de haute fréquence.

8. Analyseur de gaz suivant l'une des revendications 5 à 7, **caractérisé en ce que** le dispositif (27) d'analyse est constitué pour analyser le signal (24) de corrélation sur la base de la spectroscopie d'absorption directe.

9. Analyseur de gaz suivant l'une des revendications 5 à 8, **caractérisé**
- **en ce qu'**il y a un dispositif (10) de modulation de basse fréquence (NF), qui commande la source (8) de courant pour moduler le courant (i) supplémentairement avec une amplitude petite et au moins une fréquence supplémentaire, qui est choisie si basse qu'une modulation de longueur d'onde a lieu, et
- en ce le dispositif (27) d'analyse est constitué pour analyser le signal (24) de corrélation sur la base de la spectroscopie de modulation en longueur d'onde.
